# EUROPEAN PATENT APPLICATION

(11) **EP 4 796 062 A1**
(43) Date of publication of application: **26.08.2026**
(21) Application number: 25159319.0
(22) Date of filing: 21.02.2025
(51) Int. Cl.: A61B 18/18, A61B 18/00, A61N 5/00

(54) **LIGHT-BASED HAIR OR SKIN TREATMENT**

(71) Applicant: Koninklijke Philips N.V., 5656 AE Eindhoven (NL)
(72) Inventor: BOAMFA, Marius Iosif, 5656 AG Eindhoven (NL); CASPER, Lars Christian, 5656 AG Eindhoven (NL); ZJIROECHA, Nikolaj Vasiljevitsj, 5656 AG Eindhoven (NL); VERHAGEN, Rieko, 5656 AG Eindhoven (NL)
(74) Representative: Philips Intellectual Property & Standards

(57) **Abstract**

A device is provided for hair removal or treatment of skin. A light source is used to generate treatment light. A reflector redirects output light from the light source to an output window, and the reflector encloses a reflector volume. A contact surface is provided for contacting the skin at a front face of the contact surface. The output window forms an inner surface portion of the contact surface that closes the reflector volume, and the contact surface has an outer surface portion that extends outwardly of the reflector volume. A guide wall is used to define a cooling chamber which contacts an outside surface of the reflector and a back face of the outer surface portion of the contact surface. This cooling chamber is used to provide skin cooling as well as to draw heat away from the reflector volume.

## Description

### FIELD OF THE INVENTION

This invention relates to a device for light-based hair removal or treatment of skin.

### BACKGROUND OF THE INVENTION

For hair removal, light-based epilators are well-known. People use hair epilators or depilators to remove unwanted hair. Typical target areas for women are the face, armpit, arm, leg, and bikini line. Men use light-based epilators on the chest and back.

Light based epilators typically use Intense Pulse Light technology (IPL) to generate the treatment light. For example, a Xenon flash lamp is typically operated at a relatively low fluence, for example up to 6.5J/cm², for home-use devices. Professional devices, for permanent photo-epilation, for example may use fluences in excess of 10J/cm².

The light is absorbed by the melanin present in the hair and the hair matrix, and the generated heat damages the hair follicles. A long-lasting hair reduction result is obtained if treatment is repeated in intervals of 2 to 4 weeks.

The flash lamp operation generates heat, and this heat needs to be dissipated to prevent excessive heating of the device. It is known to use a closed light reflecting chamber to direct all light generated by the flash lamp to the treatment area. However, it is difficult to manage the heat generated by the flash lamp in a closed light reflecting chamber.

In addition to this, depending on the skin tone and the pain sensitivity of the user, the IPL pulse may be perceived as a discomfort. To address this issue, skin cooling has been used in professional saloons for permanent photo-epilation, and more recently home-use IPL devices have been developed with a skin cooling function.

The skin cooling function for home-use devices is for example implemented by active cooling of the skin contact window by means of a Peltier element, with an optional heat pipe or vapor chamber and subsequent air cooling of a system heat sink.

The presence of Peltier elements in IPL devices (for active cooling purposes of the contact window) puts energy budget constraints on the IPL device as well as cost pressures. Efficiency and cost savings would be enabled if the contact window of the IPL device could be cooled effectively by room temperature air.

US 2010/0324544 discloses an optical skin treatment system having an optical output window. An adjustment member fits over the output window, and has its own (second) output window for contacting the skin. The adjustment member is cooled by an air flow within the volume enclosed by the adjustment member together with coolant channels within the wall of the adjustment member. Alternatively, the walls of the adjustment member and the main body of the device may be hollow, and an air flow may be generated within the hollow walls.

However, these designs add significant complexity to the device.

### SUMMARY OF THE INVENTION

The invention is defined by the claims.

According to examples in accordance with an aspect of the invention, there is provided a device for hair removal or treatment of skin, comprising:
a light source for generating treatment light;
a contact surface for contacting the skin at a front of the device;
an output window through which the treatment light is delivered to a user's skin in use; and
a reflector for redirecting output light from the light source to the output window, the reflector enclosing a reflector volume,
wherein the output window forms an inner surface portion of the contact surface and closes the reflector volume,
wherein the output window forms an inner surface portion of the contact surface that closes the reflector volume,
wherein the contact surface has an outer surface portion that extends outwardly of the reflector volume, and
wherein the device comprises a guide wall for defining a cooling chamber which contacts an outside surface of the reflector and a back face opposite the outer surface portion of the contact surface.

In this device, the contact surface extends beyond the reflector, and hence beyond the reflector volume. The output window is adapted to propagate the treatment light from the reflector to the skin. A cooling chamber is formed around the outside of the reflector. The cooling chamber extends to the outside surface of the reflector and to the back of the outer portion of the contact surface. By delivering cooling air to the cooling chamber, there is cooling of the reflector volume (by cooling the outside of the reflector) and of the skin (by cooling the back of the outer surface portion). The thermal conductivity of the output window means that the cooling of the outer surface portion also implements cooling of the inner surface portion. The reflector separates the output light that is within the reflector volume from the air flow path that is within the cooling chamber.

This provides a design to provide effective cooling, using an enlarged contact surface and an air flow system to deliver air flow to the extended outer portion of the contact surface. As the cooling chamber extends to the outside surface of the reflector and to the back of the outer portion of the contact surface, the cooling chamber is able to guide cooling air around the reflector, but not through the optical path of the treatment light. This way, the cooling air does not disturb the treatment light. As a result, effective cooling is provided while maintaining effective treatment light.

In one example, the output window forms both the inner and outer surface portions. Thus, a single component may be used to define both the inner and outer surface portions. The output window for example comprises a sapphire layer.

In another example, the device further comprises an outer frame, the output window forms the inner surface portion, and the outer frame forms the outer surface portion. In this case, the output window and the surrounding part of the contact surface may be formed as different parts, each designed for their particular function.

For example, the output window may comprise a sapphire layer, and the outer frame may comprise a metal frame.

The guide wall, for example, comprises a tube that extends around the reflector. The outer surface portion closes an end of the tube. In this way, the cooling chamber comprises an annular volume between the reflector and the tube, and the annular volume is closed at the skin side (the front) by the back of the outer surface portion.

In one example, the device comprises an air flow generator adapted to generate a flow of ambient air into the cooling chamber. The flow may be delivered towards the back of the outer surface portion, and the flow leaves the cooling chamber in the opposite direction after flowing around the reflector and along the back of the outer surface portion.

The airflow generator is, for example, configured to generate an airflow to a top of the cooling chamber. The top of the cooling chamber is located opposite the contact surface. An exit flow leaves from the top of the cooling chamber.

In another example, the airflow generator is configured to generate an airflow to a bottom of the cooling chamber through openings in the guide wall. The bottom of the cooling chamber is located adjacent the contact surface. An exit flow leaves from the top of the cooling chamber. The top of the cooling chamber is located opposite the contact surface.

In another example, the device comprises an optical filter arranged in the reflector volume between the light source and the output window. For example, the optical filter is arranged in the reflector volume. The optical filter may comprise a reflective filter. The filter is used to adapt the characteristics of the light delivered to the skin.

In one example, the optical filter extends outside the reflector volume into the cooling chamber. In this way, a portion of the optical filter can be cooled within the cooling chamber. The thermal conductivity of the optical filter means that this cooling can also extract heat from the reflector volume. The optical filter for example extends to the guide wall and comprises air flow openings.

The light source for example comprises a flash lamp.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows the main functional parts of device for hair removal or treatment of skin;
Figs. 2A and 2B show known ways to provide cooling of the contact surface to the user's skin;
Fig. 3 shows a first example of some of the components of a treatment device;
Fig. 4 shows a second example of some of the components of a treatment device;
Fig. 5 shows a third example of some of the components of a treatment device;
Fig. 6 shows a fourth example of some of the components of a treatment device;
Fig. 7 shows a fifth example of some of the components of a treatment device; and
Fig. 8 shows a sixth example of some of the components of a treatment device.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for hair removal or treatment of skin. A light source is used to generate treatment light. A reflector redirects output light from the light source to an output window, and the reflector encloses a reflector volume. A contact surface is provided for contacting the skin at a front face of the contact surface. The output window forms an inner surface portion of the contact surface that closes the reflector volume, and the contact surface has an outer surface portion that extends outwardly of the reflector volume. A guide wall is used to define a cooling chamber which contacts an outside surface of the reflector and a back face of the outer surface portion of the contact surface. This cooling chamber is used to provide skin cooling as well as to draw heat away from the reflector volume.

In a setting at home, the user uses the device in contact with the user's own skin. In a commercial setting, such as a beauty salon or a spa, a professional user may use the device and place the device in contact with the skin of another person, e.g., a client of the beauty salon.

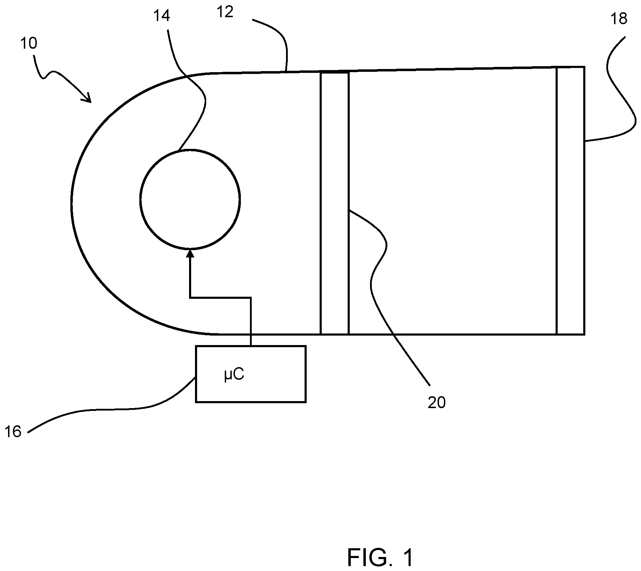

Fig. 1 shows a schematic representation of the main functional parts of a device 10 for hair removal or treatment of skin, and in particular shows an IPL device as an example. The device comprises an outer housing. A light source 14, such as a flashlamp, is arranged in the outer housing. The light source 14 is adapted to generate treatment light. A controller 16 is provided for controlling the light source 14. The light source 14 delivers treatment light to the skin of the user (and hence to hairs of the user on the skin surface) through an output window 18. The outer housing comprises a reflector 12. The internal volume enclosed by the reflector 12 and the output window 18 is referred to in this application as a reflector volume. An optical filter 20 is arranged in the reflector volume between the light source 14 and the output window 18. The optical filter 20 divides the reflector 12 into two parts - a lamp reflector 13 around the light source 14, and a tube reflector 15.

The optical filter 20 controls the optical characteristics of the light delivered to the skin. The filter for example prevents shorter wavelengths reaching the skin, as they may be harmful. The wavelengths of light are for example tuned for absorption by melanin. A reflective filter experiences reduced heating compared to an absorbing filter.

The light source 14 for example comprises a Xenon flash lamp. The flash lamp is surrounded by reflecting surfaces, including a curved rear reflector surface 12 which defines the lamp reflector 13. The curved reflector 12 shapes the light from the flashlamp into an output beam directed to the output window 18. The tube reflector part 15 ensures that all light from the Xenon flash lamp reaches a front plate which includes the output window 18, either directly or after one or more reflections.

The closed chamber formed by the reflector and filter does not allow the light source 14 to cool effectively. It is known to provide holes or a gap at the sides of the reflector to promote cooling, but high temperatures are still experienced. These thermal issues can risk breaking the optical filter 20.

Discomfort may also be experienced by the user by the high intensity light directed to the skin. The top layers of the skin absorb a small fraction of the light energy and warm up. Because these layers cannot cool efficiently and quickly, this is perceived as discomfort.

Various cooling measures are known, both for the components of the device and for the skin. This application relates in particular to skin cooling.

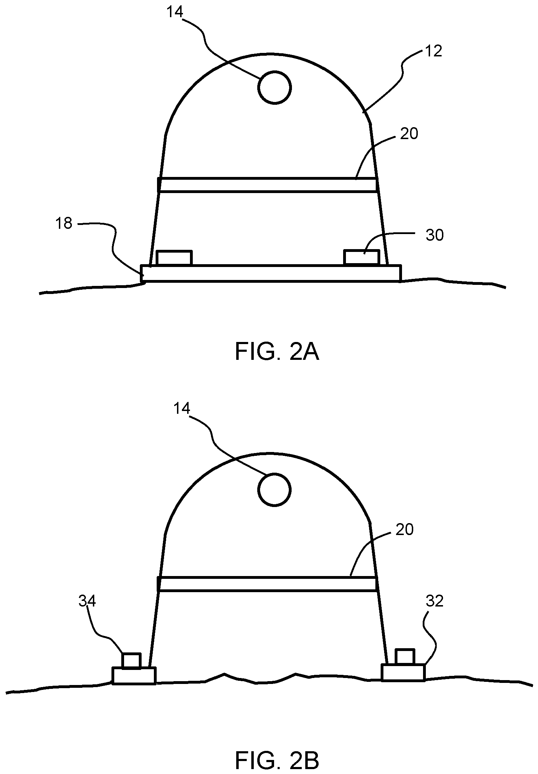

Figs. 2 to 8 show cross sections of the light source and reflector perpendicular to the light source long axis.

Fig. 2 shows two known approaches for skin cooling.

Fig. 2A shows that the output window 18 may be actively cooled by Peltier cooling elements 30. This approach provides direct cooling of the treated area, via a cooled contact window, such as a sapphire or another good thermal conductivity crystal.

Fig. 2B shows a cooled metal frame 32 that extends around the output window. This provides indirect cooling around the IPL treated area. The cooling is again implemented with active cooling elements such as Peltier elements 34.

The cold side of the Peltier cooling element is thermally connected to the output window or frame, for example using a thermal interface material.

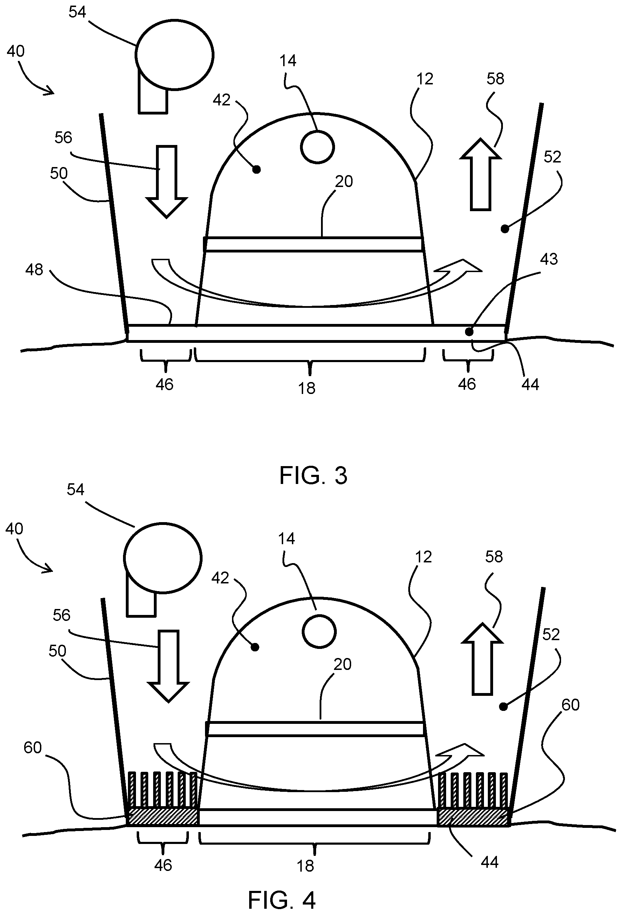

Fig. 3 shows a first example of the main functional components of a device 40 for hair removal or treatment of skin, according to the invention.

The device 40 comprises a light source 14 for generating treatment light, and an output window 18 through which the treatment light is delivered to a user's skin in use. A reflector 12 is used to redirect output light from the light source 14 to the output window 18. The reflector 12 encloses a reflector volume 42.

The output window 18 is generally made of an optically transparent material with high thermal conductivity, such as a sapphire window. Other examples of optically transparent materials with high thermal conductivity include artificial diamonds, boron nitride crystals, aluminum nitride like crystals and composite materials with graphene.

The device 40 has a contact plate 43 for contacting the skin at a front of the device 40. The contact plate 43 has a front contact surface 44 to contact the skin.

The output window 18 forms only an inner surface portion of the contact surface 44. This inner surface portion (in particular the surface opposite the inner surface portion) closes the reflector volume 42. The contact surface 44 additionally has an outer surface portion 46 that extends outwardly of the reflector volume 42.

A guide wall 50 is provided for defining a cooling chamber 52. The cooling chamber 52 is open at its top, but is closed at one side by an outside surface of the reflector 12, is closed at the front by a back face 48 of the outer surface portion 46 of the contact surface 44, and is closed at an opposite side by an inner surface of the guide wall 50. The guide wall 50 is for example part of an exterior housing wall of the device, or it may be an internal feature within the exterior housing.

The cooling chamber 52 is thus an annular shape (but not necessarily circular) around the reflector 12 and closed at the front end (the skin-side end) by the back face 48 opposite the outer surface portion 46.

An air flow generator 54, such as a pump or fan, is provided for directing a flow 56 of ambient air into the cooling chamber 52. The flow 56 is for example delivered towards the back face 48 of the contact plate 43, and the flow 56 leaves the cooling chamber in the opposite direction after flowing around the reflector 12 and along the back face 48. The exit flow is shown as 58.

The air flow 56, 58 thus does not enter the reflector volume 12 but flows around the reflector volume 42 and along the back face 48 of the contact plate 43. A high thermal conductivity of the contact plate 43 makes sure that the entire contact surface 44 is cooled, while only the back face 48 is exposed to the air flow.

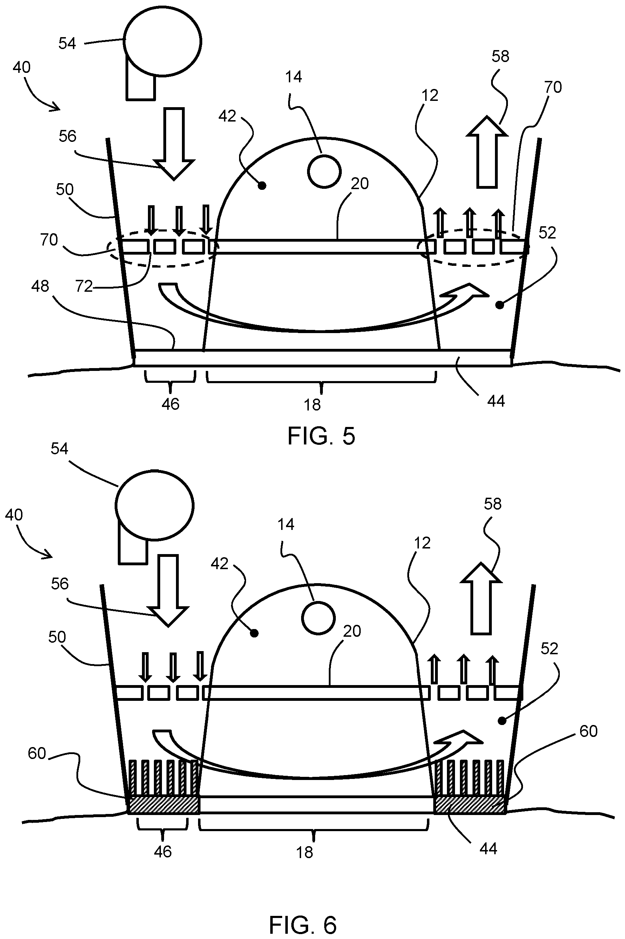

The air flow 56 may be introduced at one angular position around the cooling chamber 52, and it may exit at another angular position. For example, a cover (not shown) over the cooling chamber 52 may comprise an entrance opening and an exit opening. The height of the cover can vary, but it is preferably above the filter height and below the lamp height. The optical filter 20, for example, functions as the cover (as in Figs. 5 and 6 described below).

The air flow 56 may instead be introduced at a set of locations around the back of the annular cooling chamber 52, and it may exit the cooling chamber at another set of locations. Alternatively, there may not be a need for a cover and the air flow is simply directed towards the open back of the cooling chamber 52.

By delivering cooling air to the cooling chamber 52, the cooling air provides cooling of the reflector volume 42 by cooling the outside of the reflector 12, and the cooling air provides cooling of the skin by cooling the back face 48 of the contact plate 43. The reflector 12 separates the output light that is within the reflector volume 42 from the air flow 56, 58 within the cooling chamber 52.

In the example of Fig. 3, the output window 18 itself is extended so that it forms both the inner portion 18 and outer surface portions 46. Thus, a single component may be used to define both the inner and outer surface portions. The output window 18 for example comprises a sapphire layer.

Fig. 4 shows a second example. The same references are used as in Fig. 3 for the same components. In this design, the inner and outer surface portions of the contact surface 43 are formed by separate components. The output window 18 forms only the inner surface portion. The outer surface portion 46 is formed by an outer frame 60. The outer frame 60 is for example a frame with cooling fins 61 that extend into the cooling chamber 52. Any other high thermally conductive material may be used for the outer frame 60. The use of a separate frame may allow more effective cooling. The output window 18 may comprise a sapphire layer. The outer frame 60 may comprise a metal frame. For example, the metal frame comprises aluminum or copper.

An optical filter 20 is provided in the reflector volume 42, between the light source 14 and the output window 18. The optical filter 20 for example comprises a reflective optical filter.

Fig. 5 shows a design in which the optical filter 20 is extended into the cooling chamber 52, so that the cooling arrangement described above may also be used to cool the optical filter 20, and thereby indirectly provide additional cooling of the reflector volume 42.

The optical filter 20 extends outside the reflector volume 42 into the cooling chamber 52. Thus, an annular outer portion 70 of the optical filter 20 extends within the cooling chamber 52. A portion of the optical filter 20 is thus cooled within the cooling chamber 52. The thermal conductivity of the optical filter 20 means that this cooling can also extract heat from the reflector volume 42. The optical filter 20 comprises air flow openings 72 to allow the cooling air flow to pass to reach the back face 48.

The optical filter 20 may comprise a multi-layer structure, and all of those layers may extend outside the reflector volume 42, or only some of those layers may extend outside the reflector volume, such as a carrier substrate. The substrate of the optical filter 20 is preferably made of an optically transparent material with high thermal conductivity. By having the filter, or its substrate, extend outside of the optical path, it can be exposed to the air flow and cooled, as the substrate has good thermal conductivity. This also contributes indirectly to the thermal management of the contact surface.

Fig. 5 shows the extended optical filter applied to the design of Fig. 3.

Fig. 6 shows the extended optical filter applied to the design of Fig. 4. Thus, this design has the outer surface portion 46 formed by an outer frame 60 as well as the extended optical filter 20.

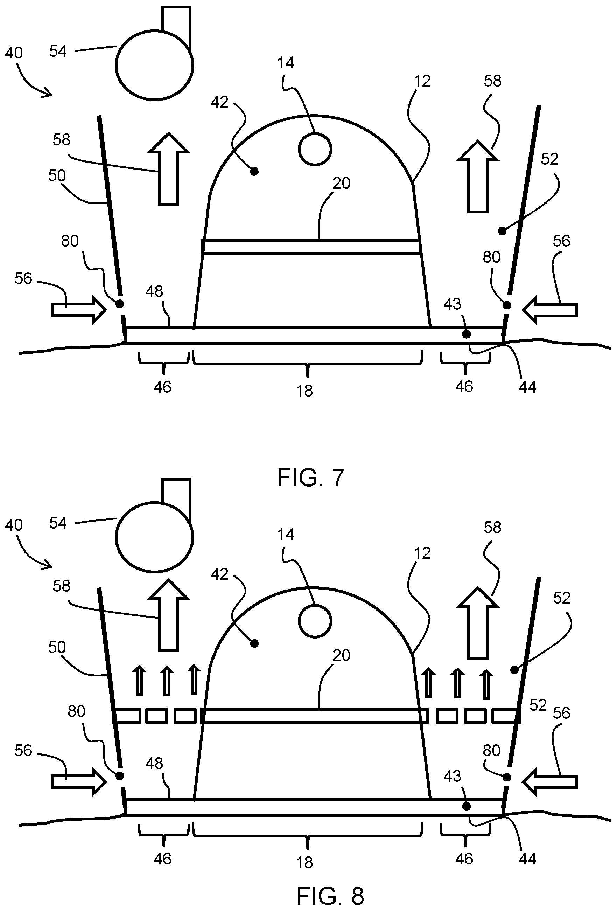

Fig. 7 shows a further example in which the guide wall 50 has openings 80 near the contact plate 43. These openings function as air entry points to the cooling chamber 52. The flow 56 of ambient air is delivered to the openings 80 or sucked in through the openings 80 by an airflow generator 54. Fig. 7 shows an implementation in which air is sucked in, rather than being pushed in and then extracted. Fig. 7 shows the change in the design of the guide wall and air flow system as applied to the configuration of Fig. 3.

Fig. 8 shows the change in the design of the guide wall and air flow system as applied to the configuration of Fig. 5. The alternative air flow arrangement of Figs. 7 and 8 may of course be applied to the configurations of Figs. 4 and 6 as well.

The air flow generator 54 is preferably internal to an exterior housing of the device 40. The exterior housing will then have an air entry port and an air exit port, and the internal walls within the exterior housing then define the desired flow passageways towards the cooling chamber 52 and away from the cooling chamber 52.

The exterior housing may be defined by an attachment which fits to an inner housing.

The invention may be applied to IPL epilators and skin treatment devices. However, it may also be applied to other (optical) epilator designs for example using LEDs or lasers. It may also be applied to other skin treatment devices using light.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

Any reference signs in the claims should not be construed as limiting the scope.

## Claims

1. A device (40) for hair removal or treatment of skin, comprising:
a light source (14) for generating treatment light;
a contact surface (44) for contacting the skin at a front of the device;
an output window (18) through which the treatment light is delivered to a user's skin in use; and
a reflector (12) for redirecting output light from the light source (14) to the output window (18), the reflector enclosing a reflector volume (42),
wherein the output window (18) forms an inner surface portion of the contact surface (44) and closes the reflector volume (42),
wherein the contact surface (44) has an outer surface portion (46) that extends outwardly of the reflector volume (42), and
wherein the device comprises a guide wall (50) for defining a cooling chamber (52) which contacts an outside surface of the reflector (12) and a back face (48) opposite the outer surface portion (46) of the contact surface (44).

2. The device of claim 1, wherein the output window (18) forms both the inner and outer surface portions.

3. The device of claim 2, wherein the output window (18) comprises a sapphire layer.

4. The device of claim 1, further comprising an outer frame (60), wherein the output window (18) forms the inner surface portion, and the outer frame (60) forms the outer surface portion (46).

5. The device of claim 4, wherein the output window (18) comprises a sapphire layer and wherein the outer frame (60) comprises a metal frame.

6. The device of any one of claims 1 to 5, wherein the guide wall (50) comprises a tube that extends around the reflector (12), wherein the outer surface portion (46) closes an end of the tube.

7. The device of any one of claims 1 to 6, further comprising an air flow generator (54) adapted to generate a flow of ambient air into the cooling chamber.

8. The device of claim 7, wherein the airflow generator is configured to generate an airflow (56) to a top of the cooling chamber,
wherein the top of the cooling chamber is located opposite the contact surface (44),
wherein an exit flow (58) leaves from the top of the cooling chamber.

9. The device of claim 7, wherein the airflow generator is configured to generate an airflow (56) to a bottom of the cooling chamber through openings (70) in the guide wall (50),
wherein the bottom of the cooling chamber is located adjacent the contact surface (44),
wherein an exit flow (58) leaves from the top of the cooling chamber,
wherein the top of the cooling chamber is located opposite the contact surface (44).

10. The device of any one of claims 1 to 9, further comprising an optical filter (20) arranged in the reflector volume (42) between the light source (14) and the output window (18).

11. The device of claim 10, wherein the optical filter (20) extends outside the reflector volume into the cooling chamber (52).

12. The device of claim 11, wherein the optical filter extends to the guide wall (50) and comprises air flow openings (72).

13. The device of any one of claims 1 to 12, wherein the light source (14) comprises a flash lamp.
